# EUROPÄISCHE PATENTSCHRIFT

(11) **EP 0 690 060 B1**
(45) Veröffentlichungstag und Bekanntmachung des Hinweises auf die Patenterteilung: **09.09.1998**
(21) Anmeldenummer: 95109777.3
(22) Anmeldetag: 23.06.1995
(51) Int. Cl.: C07D 498/10, C07F 7/08, C07F 7/18, C07F 9/6574, C08K 5/353

(54) **Polyalkylpiperidin-Verbindungen**
Polyalkylpiperidine compounds
Composés de polyalkylpipéridine

(30) Priorität: 01.07.1994 DE 4423054
(43) Veröffentlichungstag der Anmeldung: 03.01.1996
(73) Patentinhaber: Clariant GmbH, 65929 Frankfurt am Main (DE)
(72) Erfinder: Gaa, Karl, Dr., D-89349 Burtenbach (DE); Zäh, Matthias, Dr., D-86368 Gersthofen (DE)

(56) Entgegenhaltungen:
- EP-A- 0 057 885

## Beschreibung

Die vorliegende Erfindung bezieht sich auf neue Polyalkylpiperidin-Verbindungen, die sich durch besonders niedrige Flüchtigkeit in der Wärme auszeichnen.

Es ist bekannt, daß organische Verbindungen durch Licht, Strahlung, Sauerstoff oder Wärme geschädigt werden. Es gibt bereits viele Druckschriften, die Verbindungen zur Stabilisierung von organischem Material beschreiben. Ein Teil davon betrifft Verbindungen auf der Basis des 2,2,6,6-Tetramethylpiperidins. Speziell Spiro-Verbindungen haben sich als besonders wirksam erwiesen (vgl. EP 8 102, EP 28 318, EP 208 263).

Weiterhin sind Diazaspirodecane der Formel 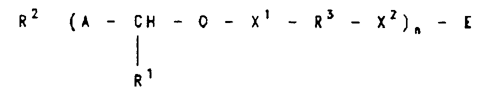 worin R¹ eine Diazaspirodecan-Gruppe darstellt, bekannt (vgl. EP 57 885). Als Nachteil dieser Verbindungen hat sich herausgestellt, daß sie in der Wärme relativ flüchtig sind.

Überraschenderweise wurde gefunden, daß Verbindungen mit ähnlicher Struktur eine deutlich geringere Flüchtigkeit aufweisen.

Die Erfindung betrifft daher Verbindungen der Formel I 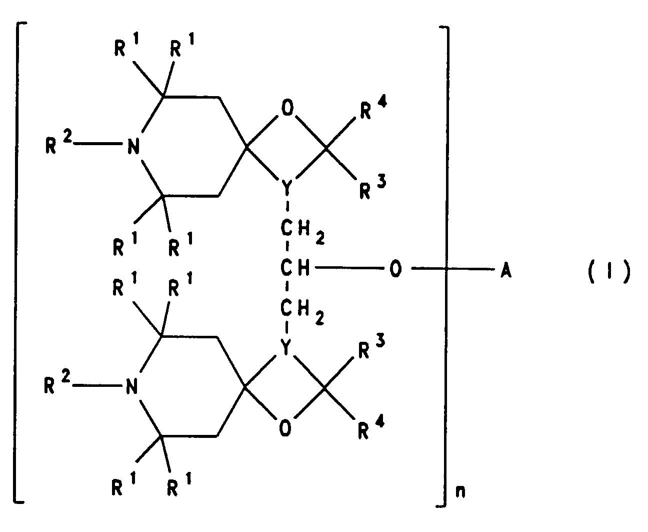 worin Y eine der Gruppen 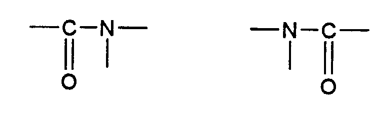 ist,
R¹ ein Wasserstoffatom oder eine Alkylgruppe bedeutet,
R² ein Wasserstoffatom oder eine Alkylgruppe ist,
R³ und R⁴ gleich oder verschieden sind und ein Wasserstoffatom oder eine Alkylgruppe bedeuten, oder R³ und R⁴ zusammen mit dem sie verbindenden C-Atom einen Ring mit 5 bis 12 Ringliedern bilden,
n = 1, 2 oder 3 bedeutet und
A bei n = 1
-COOR⁵, -P(OR⁶)(OR⁷), -Si(OR⁵)(OR⁵)(OR⁵), -SiR⁵R⁵R⁵ oder die Gruppe 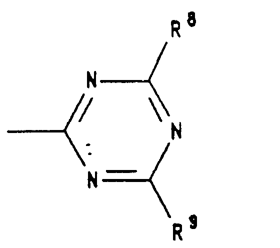 A bei n = 2
-CO-, =POR⁵, =Si(OR⁵)(OR⁵), =SiR⁵R⁵ oder die Gruppe 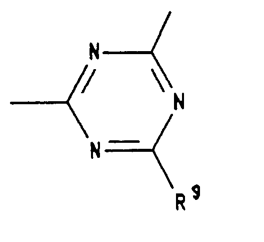 A bei n = 3
≡Si(OR⁵), ≡SiR⁵ oder die Gruppe 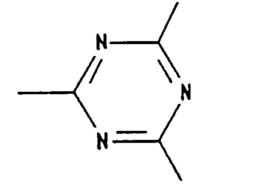 bedeutet,
R⁵ eine C₁ - C₁₈ -Alkylgruppe, eine C₂ - C₁₈ -Alkenylgruppe, eine C₂ - C₁₈ - Alkinylgruppe, eine C₅ - C₁₂ -Cycloalkylgruppe, eine C₆ - C₁₀ -Bicycloalkylgruppe, eine C₅ - C₈ -Cycloalkenylgruppe, eine C₆ - C₁₀ -Arylgruppe, eine C₇ - C₉ - Aralkylgruppe, eine C₇ - C₉ -Alkarylgruppe, gegebenenfalls substituiert durch C₁ - C₄ -Alkyl oder Phenyl, bedeutet, wobei mehrere Reste R⁵ gleich oder verschieden sein können,
R⁶ und R⁷ gleich oder verschieden sind und eine C₁ - C₁₈ -Alkylgruppe, eine C₂ - C₁₈ -Alkenylgruppe, eine C₂ - C₁₈ -Alkinylgruppe, eine C₅ - C₁₂ -Cycloalkylgruppe, eine C₆ - C₁₀ -Bicycloalkylgruppe, eine C₅ - C₈ -Cycloalkenylgruppe, eine C₆ - C₁₀ - Arylgruppe, eine C₇ - C₉ -Aralkylgruppe oder eine C₇ - C₉ -Aralkylgruppe, substituiert durch C₁ - C₄ -Alkyl oder Phenyl, bedeuten, oder ein Rest sind der Formel II oder III 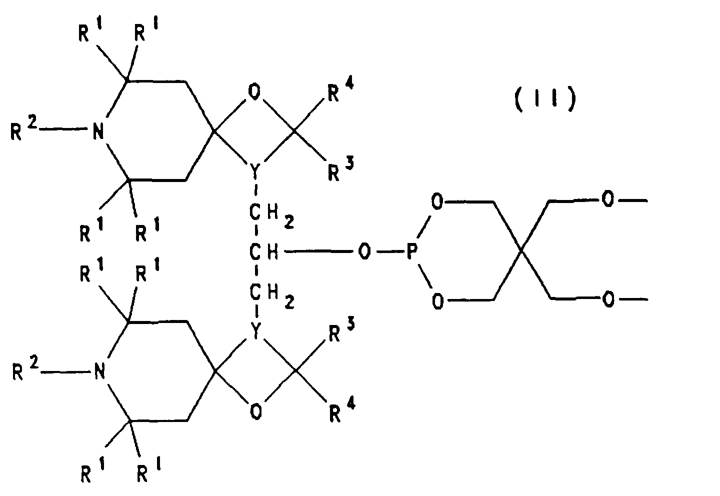 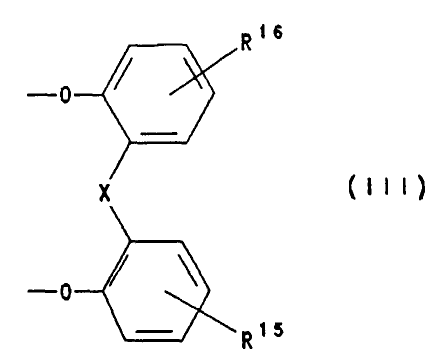 worin R¹, R², R³, R⁴ und R⁵ die obengenannte Bedeutung haben,
und X eine direkte Bindung oder Methylengruppe oder eine 1,1-Alkylidengruppe mit 2 bis 5 C-Atomen bedeutet,
R⁸ und R⁹ gleich oder verschieden sind und -OR⁵, NHR⁵, -NR¹¹R¹², oder eine der beiden Gruppen 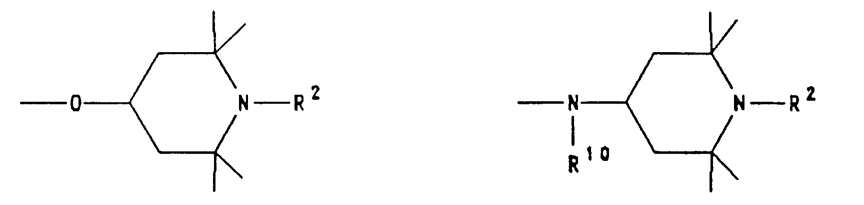 worin R¹⁰ ein Wasserstoffatom, eine C₁ - C₁₂ -Alkylgruppe, eine C₅ - C₇ - Cycloalkylgruppe, eine C₇ - C₉ -Aralkylgruppe, eine C₈ - C₁₈ -Alkanoylgruppe, eine C₃ - C₅ -Alkenoylgruppe oder eine Benzoylgruppe bedeutet, oder 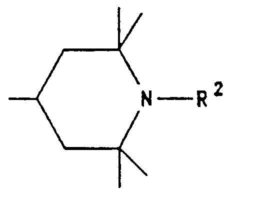 ist,
R¹¹ und R¹² gleich oder verschieden sind und eine C₁ - C₁₀ -Alkylgruppe, eine C₅ - C₇ -Cycloalkylgruppe oder eine C₆ - C₁₀ -Arylgruppe bedeuten, oder R¹¹ und R¹² zusammen mit dem Stickstoff einen 5- bis 12-gliedrigen Ring bilden, der auch noch Sauerstoff enthalten kann.

Die Erfindung bezieht sich auch auf die Herstellung dieser Verbindungen sowie die Verwendung zum Stabilisieren von organischem Material, insbesondere von Kunststoffen, Ölen und Lacken.

In der Formel I 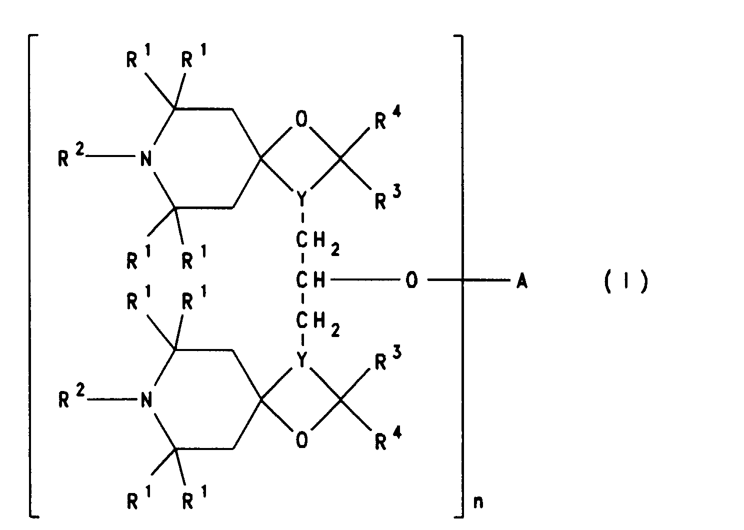 ist Y eine der Gruppen

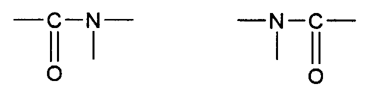

Bevorzugt ist Y eine Gruppierung, bei welcher das Sauerstoffatom dem die beiden Ringe gemeinsamen C-Atom benachbart ist, dh. 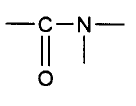 n ist 1, 2 oder 3, vorzugsweise 1.

R¹ ist ein Wasserstoffatom oder eine C₁ - C₄ -Alkylgruppe, vorzugsweise Methylgruppe.

R² ist ein Wasserstoffatom oder eine C₁ - C₁₂ -Alkylgruppe, vorzugsweise Methylgruppe.

R³ und R⁴ sind gleich oder verschieden und bedeuten ein Wasserstoffatom, eine C₁ - C₈ - , vorzugsweise C₁ - C₄ -Alkylgruppe oder R³ und R⁴ bilden zusammen mit dem sie verbindenden C-Atom einen Ring mit 5 bis 12, vorzugsweise 6 oder 12 Ringgliedern. Vorzugsweise bedeuten R³ und R⁴ Methyl, Sechsring oder Zwölfring.

R⁵ ist eine C₁ - C₁₈ -, vorzugsweise C₁ - C₈ -Alkylgruppe, eine C₂ - C₁₈ - vorzugsweise C₃ - C₆ -Alkenylgruppe, eine C₂ - C₁₈ -, vorzugsweise C₂ - C₈ - Alkinylgruppe, eine C₅ - C₁₂ -, vorzugsweise C₅ - C₆ -Cycloalkylgruppe, eine C₆ - C₁₀ vorzugsweise C₆ - C₁₀ -Bicycloalkylgruppe, eine C₅ - C₈ -, vorzugsweise C₅ - C₆ - Cycloalkenylgruppe, eine C₆ - C₁₀ -, vorzugsweise C₆- oder C₁₀ -Arylgruppe, eine C₇ - C₉ -Aralkylgruppe, eine C₇ - C₉ -Aralkylgruppe, substituiert durch C₁ - C₄ -Alkyl Phenyl, wobei mehrere Reste R⁵ gleich oder verschieden sein können,
Bei n = 1 ist A eine Gruppe -COOR⁵, -P(OR⁶)(OR⁷), -Si(OR⁵)(OR⁵)(OR⁵) oder -SiR⁵R⁵R⁵, worin R⁵ die obengenannte Bedeutung hat.

R⁶ und R⁷ sind gleich oder verschieden und bedeuten eine C₁ - C₁₈ -, vorzugsweise C₁ - C₈ -Alkylgruppe, eine C₂ - C₁₈ -, vorzugsweise C₃ - C₆ -Alkenylgruppe, eine C₂ - C₁₈ -, vorzugsweise C₂ - C₈ -Alkinylgruppe, eine C₅ - C₁₂ -, vorzugsweise C₅ - C₆ - Cycloalkylgruppe, eine C₆ - C₁₀ -, vorzugsweise C₈ - C₁₀ -Bicycloalkylgruppe, eine C₅ - C₈ -, vorzugsweise C₅ - C₆ -Cycloalkenylgruppe, eine C₆ - C₁₀ -, vorzugsweise C₆- - oder C₁₀ -Arylgruppe, eine C₇ - C₉ -Aralkylgruppe oder eine C₇ - C₉ - Aralkylgruppe, substituiert durch C₁ - C₄ -Alkyl oder Phenyl, oder sind ein Rest der Formel II oder III 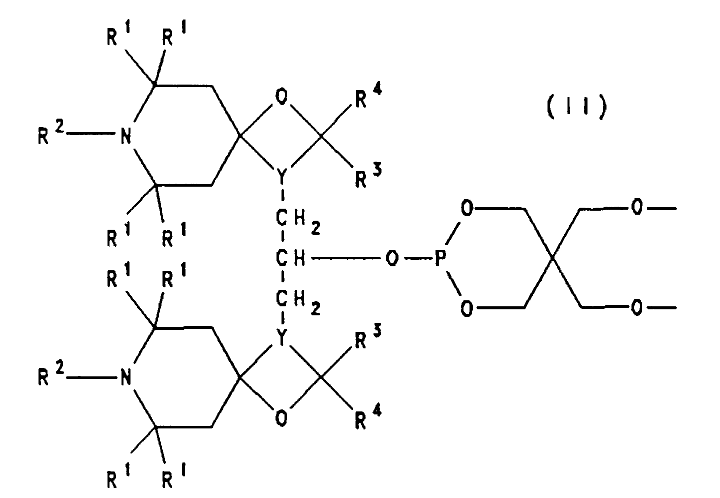 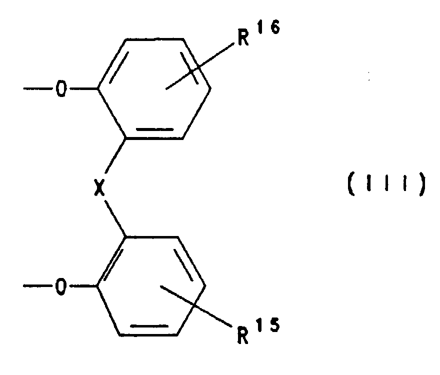 worin R¹, R², R³, R⁴ und R⁵ die obengenannte Bedeutung haben, und X eine direkte Bindung oder Methylengruppe oder eine 1,1-Alkylidengruppe mit 2 bis 5 C-Atomen bedeutet.
Oder A ist 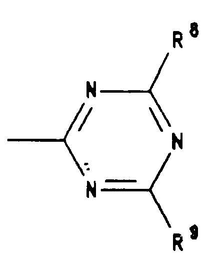 worin R⁸ und R⁹ gleich oder verschieden sind und -OR⁵, -NHR⁵, -NR¹¹R¹², oder eine der beiden Gruppen 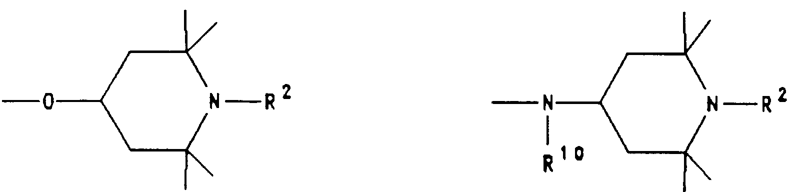 bedeuten, worin R¹⁰ ein Wasserstoffatom, eine C₁ - C₁₂ -, vorzugsweise C₁ - C₈ - Alkylgruppe, eine C₅ - C₇ -, vorzugsweise C₅ - C₆ -Cycloalkylgruppe, eine C₇ - C₉ - Aralkylgruppe, eine C₆ - C₁₈ -, vorzugsweise C₂ - C₈ -Alkanoylgruppe, eine C₃ - C₅ - vorzugsweise C₃ - C₄ -Alkenoylgruppe oder eine Benzoylgruppe bedeutet oder 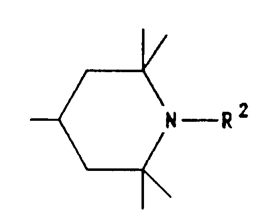 ist.

R¹¹ und R¹² sind gleich oder verschieden und bedeuten eine C₁ - C₁₈-, vorzugsweise C₁ - C₈ -Alkylgruppe, eine C₅ - C₇ -Cycloalkylgruppe, eine C₆ - C₁₀ -, vorzugsweise C₆ -Arylgruppe, oder R¹¹ und R¹² bilden zusammen mit dem Stickstoff einen 5- bis 12-, vorzugsweise 5- bis 6-gliedrigen Ring, der auch noch Sauerstoff enthalten kann, insbesondere einen Pyrrolidin-, Piperidin- oder Morpholinring.

Vorzugsweise ist A -Si(CH₃)₃, -COO-Alkyl oder 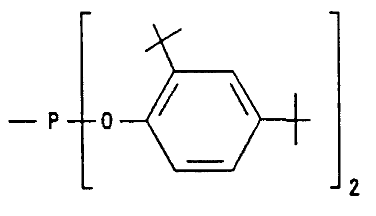 Bei n = 2 ist A eine Gruppe -CO-, =POR⁵, =Si(OR⁵)(OR⁵) oder =SiR⁵R⁵ oder die Gruppe 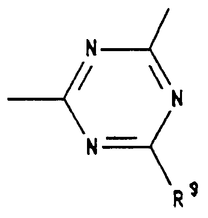 worin R⁵ und R⁹ die obengenannte Bedeutung haben.

Vorzugsweise ist A -CO-, -Si(CH₃)₃, 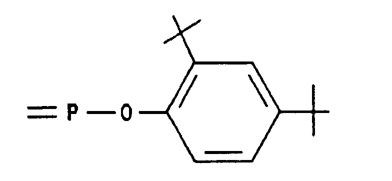 oder 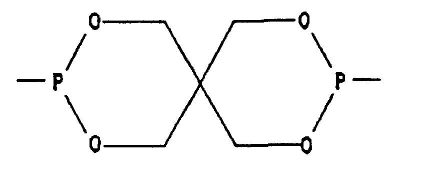 Bei n = 3 ist A ≡Si(OR⁵), ≡SiR⁵ oder die Gruppe 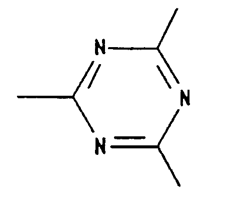 wobei R⁵ die obengenannte Bedeutung hat.

Vorzugsweise ist A

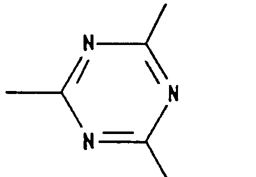

Die Verbindungen der Formel I mit A nicht H lassen sich vorteilhafterweise durch an sich bekannte Umsetzungen aus den Alkoholen der Formel IV 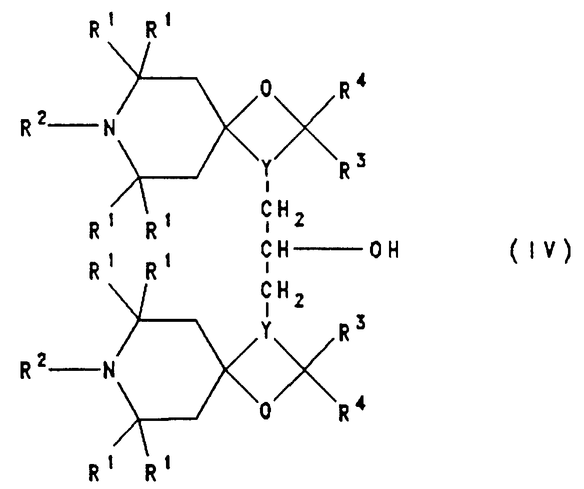 mit den entsprechenden reaktiven Komponenten der Formel V 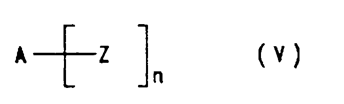 herstellen, wobei
Z ein Halogenatom oder die Gruppe -OR⁵ bedeutet für A = -CO-, -COOR⁵, -P(OR⁶)(OR⁷), =POR⁵;
und Z ein Halogenatom ist für A = -Si(OR⁵)(OR⁵)(OR⁵), SiR⁵R⁵R⁵, =Si(OR⁵)(OR⁵), =SiR⁵R⁵, ≡Si(OR⁵), ≡SiR⁵ oder ein Triazinderivat.
R⁵, R⁶ und R⁷ haben die obengenannte Bedeutung.

Eine weitere Möglichkeit zur Herstellung der erfindungsgemäßen Verbindungen der Formel I mit R² = Alkyl besteht darin, die Produkte I mit R² = H mit den üblichen Reagenzien zu den substituierten Produkten I mit R² = Alkyl umzusetzen.

Die Umsetzung erfolgt in einem protischen oder aprotischen, organischen Lösemittel, vorzugsweise einem Kohlenwasserstoff, insbesondere einem aromatischen Kohlenwasserstoff wie beispielsweise Toluol, Xylol oder in Gemischen hieraus oder in einem Alkohol, vorzugsweise einem aliphatischen Alkohol, insbesondere in einem C₁-C₁₂-Alkohol wie in Isopropanol oder in einer Mischung aus einem Kohlenwasserstoff und einem Alkohol, insbesondere Xylol und Isopropanol. Eine weitere Möglichkeit besteht darin, eine der Reaktionskomponenten im Überschuß als Lösemittel zu verwenden.

Die Umsetzung erfolgt in Gegenwart von Basen. Eingesetzt werden molare Mengen an Base, wobei für Umesterungen auch katalytischen Mengen ausreichend sind.

Die Umsetzung erfolgt bei einer Temperatur zwischen 20°C und dem Siedepunkt des Lösemittels, die geeignete Temperatur ist abhängig von der verwendeten Base und von der Reaktivität der eingesetzten Verbindung der Formel V.

Die erfindungsgemäßen Verbindungen der Formel I können in Form der freien Basen oder als Säureadditionssalz vorliegen. Geeignete Anionen stammen beispielsweise von anorganischen Säuren und insbesondere von organischen Säuren oder Sulfonsäuren. Als anorganische Anionen sind beispielsweise Chlorid, Bromid, Sulfat, Tetrafluoroborat, Phosphat und Rhodanid zu nennen. Als Carbonsäuren kommen Formiat, Acetat, Propionat, Hexanoat, Cyclohexanoat, Lactat, Stearat, Acrylat, Methacrylat, Citrat, Malonat oder Succinat sowie Anionen von Polycarbonsäuren mit bis zu 3000 COOH-Gruppen in Betracht. Sulfonsäure-Anionen sind beispielsweise Benzolsulfonat oder Tosylat.

Die erfindungsgemäßen Verbindungen eignen sich in hervorragender Weise zum Stabilisieren von organischen Material gegen die Einwirkung von Licht, Sauerstoff und Wärme. Sie werden dem zu stabilisierenden organischen Material in einer Konzentration von 0.001 bis 5 Gew.-%, vorzugsweise von 0.02 bis 1 Gew.-%, bezogen auf das organische Material vor, während oder nach seiner Herstellung zugesetzt.

Unter organischem Material sind beispielsweise Vorprodukte für Kunststoffe, Lacke und Öle, insbesondere jedoch Kunststoffe, Lacke und Öle selbst, zu verstehen.

Gegenstand der vorliegenden Erfindung ist außerdem gegen die Einwirkung von Licht, Sauerstoff und Wärme stabilisiertes organisches Material, insbesondere Kunststoffe, Lacke und Öle, welches die Verbindung in den oben angegebenen Konzentrationen enthält. Zu diesen organischen Materialien gehören folgende Stoffe:
1. Polymere von Mono- und Diolefinen, beispielsweise Polyethylen hoher, mittlerer oder niederer Dichte (das gegebenenfalls vernetzt sein kann), Polypropylen, Polyisobutylen, Polybuten-1, Polymethylpenten-1, Polyisopren oder Polybutadien sowie Polymerisate von Cycloolefinen, wie beispielsweise von Cyclopenten oder Norbornen.
2. Mischungen der unter 1) genannten Polymeren, beispielsweise Mischungen von Polypropylen mit Polyethylen oder mit Polyisobutylen.
3. Copolymere von Mono- und Diolefinen untereinander oder mit anderen Vinylmonomeren, wie beispielsweise Ethylen-Propylen-Copolymere, Propylen-Buten-1-Copolymere, Propylen-lsobutylen-Copolymere, Ethylen-Buten-1-Copolymere, Propylen-Butadien-Copolymere, Isobutylen-lsopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (lonomere), sowie Terpolymere von Ethylen mit Propylen und einem Dien, wie Hexadien, Dicyclopentadien oder Ethylidennorbornen.
4. Polystyrol, Poly(p-methylstyrol).
5. Copolymere von Styrol oder α-Methylstyrol mit Dienen oder Acrylderivaten, wie beispielsweise Styrol-Butadien, Styrol-Maleinsäureanhydrid, Styrol-Acrylnitril, Styrol-Ethylmethacrylat, Styrol-Butadien-Ethylacrylat, Styrol-Acrylnitril-Methacrylat; Mischungen von hoher Schlagzähigkeit aus Styrol-Copolymeren und einem anderen Polymer, wie beispielsweise einem Polyacrylat, einem Dien-Polymeren oder einem Ethylen-Propylen-Dien-Terpolymeren; sowie Block-Copolymere des Styrols, wie beispielsweise Styrol-Butadien-Styrol, Styrol-lsopren-Styrol, Styrol-Ethylen/Butylen-Styrol oder Styrol-Ethylen/Propylen-Styrol.
6. Pfropfcopolymere von Styrol, wie beispielsweise Styrol auf Polybutadien, Styrol und Acrylnitril auf Polybutadien, Styrol und Maleinsäureanhydrid auf Polybutadien, Styrol und Alkylacrylate oder Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, die beispielsweise als sogenannte ABS-, MBS-, ASA- oder AES-Polymere bekannt sind.
7. Polyvinylchlorid.
8. Mischpolymerisate des Vinylchlorids, welche durch die bekannten Verfahren hergestellt werden können (beispielsweise Suspensions-, Masse- oder Emulsionspolymerisation).
9. Mischpolymere des Vinylchlorids mit bis zu 30 Gew.-% an Comonomeren, wie beispielsweise Vinylacetat, Vinylidenchlorid, Vinylether, Acrylnitril, Acrylsäureester, Maleinsäuremono- oder -diester oder Olefinen, sowie Pfropfpolymerisate des Vinylchlorids.
10. Halogenhaltige Polymere, wie beispielsweise Polychloropren, Chlorkautschuk, chloriertes oder chlorsulfoniertes Polyethylen, Epichlorhydrin-homo- und - copolymere, insbesondere Polymere aus halogenhaltigen Vinylverbindungen, wie beispielsweise, Polyvinylidenchlorid, Polyvinylfluorid, Polyvinylidenfluorid; sowie deren Copolymere wie von Vinylchlorid-Vinylidenchlorid, Vinylchlorid-Vinylacetat oder Vinylidenchlorid-Vinylacetat.
11. Polymere, die sich von α-β-ungesättigten Säuren und deren Derivaten ableiten, wie Polyacrylate und Polymethacrylate, Polyacrylamide und Polyacrylnitrile.
12. Copolymere der unter 11) genannten Monomeren untereinander oder mit anderen ungesättigten Monomeren, wie beispielsweise Acrylnitril-Butadien-Copolymere, Acrylnitril-Alkylacrylat-Copolymere, Acrylnitril-Alkoxyacrylat-Copolymere, Acrylnitril-Vinylhalogenid-Copolymere oder Acrylnitril-Alkylmethacrylat-Butadien-Copolymere.
13. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.
14. Homo- und Copolymere von cyclischen Ethern, wie Polyethylenglykole, Polyethylenoxyd, Polypropylenoxyd oder deren Copolymere mit Bisglycidylethern.
15. Polyacetale, wie Polyoxymethylen, sowie solche Polyoxymethylene, die Comonomere wie beispielsweise Ethylenoxyd enthalten.
16. Polyphenylenoxyde und -sulfide und deren Mischungen mit Styrolpolymeren.
17. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte (Polyisocyanate-Polyole-Prepolymere).
18. Polyamide und Copolyamide, die sich von Diaminen und Dicarbonsäuren und/oder von Aminocarbonsäuren oder den entsprechenden Lactamen ableiten, wie Polyamid-4, Polyamid-6, Polyamid-6.6, Polyamid-6.10, Polyamid-11, Polyamid-12, Poly-2,4,4-trimethylhexamethylenterephthalamid, Poly-m-phenylenisophthalamid, sowie deren Copolymere mit Polyethern, wie beispielsweise mit Polyethylenglykol, Polypropylenglykol oder Polytetramethylenglykol.
19. Polyharnstoffe, Polyimide und Polyamid-imide.
20. Polyester, die sich von Dicarbonsäuren und Diolen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-(2,2-bis-(4-hydroxyphenyl)-propan)-terephthalat, Polyhydroxybenzoate, sowie Block-Polyether-ester, die sich von Polyethylen mit Hydroxyendgruppen, Dialkoholen und Dicarbonsäuren ableiten.
21. Polycarbonate und Polyestercarbonate.
22. Polysulfone, Polyethersulfone und Polyetherketone.
23. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
24. Trocknende und nicht trocknende Alkydharze.
25. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
26. Vernetzbare Acrylharze, die sich von substituierten Acrylsäureestern ableiten, wie beispielsweise Epoxyacrylaten, Urethan-acrylaten oder Polyester-acrylaten.
27. Alkydharze, Polyesterharze und Acrylatharze, die mit Melaminharzen, Harnstoffharzen, Polyisocyanaten oder Epoxidharzen vernetzt sind.
28. Vernetzbare Epoxidharze, die sich von Polyepoxiden ableiten, beispielsweise von Bis-glycidylethern oder von cycloaliphatischen Diepoxiden.
29. Natürliche Polymere, wie Cellulose, Naturkautschuk, Gelatine sowie deren polymerhomolog chemisch abgewandelte Derivate, wie Celluloseacetate, -propionate und -butyrate, oder Celluloseether, wie Methylcellulose.
30. Mischungen der oben erwähnten Polymeren, wie beispielsweise PP/EPDM, Polyamid-6/EPDM oder ABS, PVC/EVA, PVC/ABS, PVC/MBS, PC/ABS, PBTP/ABS, PC/ASA, PC/PBT, PVC/CPE, PVD/Acrylat, POM/thermoplastisches PUR, POM/Acrylat, POM/MBS, PPE/HIPS, PPE/Polyamid-6.6 und Copolymere, PA/HDPE, PA/PP, PA/PPE.
31. Natürlich vorkommende und synthetische organische Stoffe, welche reine Monomere oder Mischungen von Monomeren sind, wie beispielsweise Mineralöle, tierische und pflanzliche Fette, Öle und Wachse, oder Öle, Fette und Wachse auf Basis synthetischer Ester oder Mischungen dieser Stoffe.
32. Wäßrige Dispersionen von Natur- oder Synthesekautschuk.

Bevorzugt werden mit den erfindungsgemäßen Verbindungen Polyolefine wie Polyethylen und Polypropylen stabilisiert.

Das durch die erfindungsgemäßen Verbindungen stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen zugesetzt werden können, sind beispielsweise Verbindungen auf der Basis sterisch gehinderter Amine oder sterisch gehinderter Phenole oder Schwefel oder Phosphor enthaltende Costabilisatoren.

Als derartige Verbindungen kommen beispielsweise in Frage:
1. Antioxidantien
   1.1 Alkylierte Monophenole, beispielsweise 2,6 Di-t-butyl-4-methylphenol, 2-Butyl-4,6-dimethylphenol, 2,6-Di-t-butyl-4-ethylphenol, 2,6-Di-t-butyl-4-n-butylphenol, 2,6-Di-t-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tricyclohexylphenol, 2,6-Di-t-butyl-4-methoxymethylphenol, 2,6-Di-nonyl-4-methylphenol, 2,4-Dimethyl-6(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen dieser Verbindungen.
   1.2 Alkylthiomethylphenole, beispielsweise 2,4-Di-octylthiomethyl-6-t-butylphenol, 2,4-Dioctylthiomethyl-6-methylphenol, 2,4-Di-octylthiomethyl-6-ethylphenol, 2,6-Di-dodecylthiomethyl-4-nonylphenol.
   1.3 Hydrochinone und alkylierte Hydrochinone, beispielsweise 2,6-Di-t-butyl-4-methoxyphenol, 2,5-Di-t-butyl-hydrochinon, 2,5-Di-t-amyl-hydrochinon, 2,6-Diphenyl-4-octadecyloxyphenol, 2,6-Di-t-butyl-hydrochinon, 2,5-Di-t-butyl-4-hydroxyanisol, 3,5-Di-t-butyl-4-hydroxyanisol, 3,5-Di-t-butyl-4-hydroxyphenolstearat, Bis-(3,5-di-t-butyl-4-hydroxyphenyl)adipat.
   1.4 Hydroxylierte Thiodiphenylether, beispielsweise 2,2'-Thio-bis-(6-t-butyl-4-methylphenol), 2,2'-Thio-bis-(4-octylphenol), 4,4'-Thio-bis-(6-t-butyl-3-methylphenol), 4,4'-Thio-bis-(6-t-butyl-2-methyl-phenol), 4,4'-Thio-bis-(3,6-di-sec.-amylphenol), 4,4'-Bis-(2,6-dimethyl-4-hydroxyphenyl)-disulfid.
   1.5 Alkyliden-Bisphenole, beispielsweise 2,2'-Methylen-bis-(6-t-butyl-4-methylphenol), 2,2'-Methylen-bis-(6-t-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylenbis-(6-nonyl-4-methylphenol), 2,2'-Methylen-bis-(4,6-di-t-butylphenol), 2,2'-Ethyliden-bis-(4,6-di-t-butyiphenol), 2,2'-Ethyliden-bis-(6-t-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], -4-nonylphenol], 4,4'-Methylen-bis-(2,6-di-t-butylphenol), 4,4'-Methylen-bis-(6-t-butyl-2-methylphenol), 1,1-Bis-(5-t-butyl-4-hydroxy-2-methylphenyl)-butan, 2,6-Bis-(3-t-butyl-5-methyl-2-hydroxybenzyl)-4-methylphenol, 1,1,3-Tris-(5-t-butyl-4-hydroxy-2-methyl-phenyl)-butan, 1,1-Bis-(5-t-butyl-4-hydroxy-2-methylphenyl)-3-n-dodecylmercaptobutan, Bis-(3-t-butyl-4-hydroxy-5-methyl-phenyl)-dicyclopentadien, Bis-[2-(3'-t-butyl-2'-hydroxy-5'-methylbenzyl)-6-t-butyl-4-methyl-phenyl]-terephthalat, 1,1-Bis-(3,5-dimethyl-2-hydroxyphenyl)-butan, 2,2-Bis-(3,5-di-t-butyl-4-hydroxyphenyl)-propan, 2,2-Bis-(5-t-butyl-4-hydroxy-2-methylphenyl)-4-n-dodecylmercaptobutan, 1,1,5,5-Tetra-(5-t-butyl-4-hydroxy-2-methylphenyl)-pentan.
   1.6 O-, N- und S-Benzylverbindungen, beispielsweise 3,5,3',5'-Tetra-t-butyl-4,4'-dihydroxydibenzylether, Octadecyl-4-hydroxy-3,5-dimethylbenzyl-mercaptoacetat, Tris-(3,5-di-t-butyl-4-hydroxybenzyl)-amin, Bis-(4-t-butyl-3-hydroxy-2,6-dimethylbenzyl)-di-thioterephthalat, Bis-(3,5-di-t-butyl-4-hydroxybenzyl)-sulfid, Isooctyl-3,5-di-t-butyl-4-hydroxybenzyl-mercaptoacetat.
   1.7 Hydroxybenzylierte Malonate, beispielsweise Dioctadecyl-2,2-bis-(3,5-di-t-butyl-2-hydroxybenzyl)-malonat, Di-octadecyl-2-(3-t-butyl-4-hydroxy-5-methylbenzyl)-malonat, Didodecylmercaptoethyl-2,2-bis-(3,5-di-t-butyl-4-hydroxybenzyl)-malonat.
   1.8 Hydroxybenzyl-Aromaten, beispielsweise 1,3,5-Tris-(3,5-di-t-butyl-4-hydroxybenzyl)-2,4,6-trimethylbenzol, 1,4-Bis-(3, 5-di-t-butyl-4-hydroxybenzyl)2,3,5,6-tetramethylbenzol, 2,4,6-Tris-(3,5-di-t-butyl-4-hydroxybenzyl)-phenol.
   1.9 Triazinverbindungen, beispielsweise 2,4-Bis-octylmercapto-6-(3,5-di-t-butyl-4-hydroxyanilino)-1,3,5-triazin, 2-Octylmercapto-4,6-bis-(3,5-di-t-butyl-4-hydroxyanilino)-1 ,3, 5-triazin, 2-Octylmercapto-4, 6-bis-(3, 5-di-t-butyl-4-hydroxyphenoxy)-1,3,5-triazin, 2,4,6-Tris-(3,5-di-t-butyl-4-hydroxyphenoxy)-1,2,3-triazin, 1,3,5-Tris-(3,5-t-butyl-4-hydroxybenzyl)-isocyanurat, 1 ,3,5-Tris-(4-t-butyl-3-hydroxy-2,6-dimethylbenzyl)-isocyanurat, 2,4,6-Tris-(3,5-di-t-butyl-4-hydroxyphenylethyl)-1,3,5-triazin, 1,3,5-Tris-(3,5-di-t-butyl-4-hydroxyphenylpropionyl)-hexahydro-1,3;5-triazin, 1,3,5-Tris-(3,5-dicyclohexyl-4-hydroxybenzyl)-isocyanurat.
   1.10 Benzylphosphonate, beispielsweise Dimethyl-2,5-di-t-butyl-4-hydroxybenzylphosphonat, Diethyl-3,5-di-t-butyl-4-hydroxybenzylphosphonat, Dioctadecyl-3,5-di-t-butyl-4-hydroxybenzylphosphonat, Dioctadecyl-5-t-butyl-4-hydroxy-3-methylbenzylphosphonat, Ca-Salz des 3,5-di-t-butyl-4-hydroxybenzylphosphonsäure-monoethylesters.
   1.11 Acylaminophenole, beispielsweise 4-Hydroxy-laurinsäureanilid, 4-Hydroxystearinsäureanilid, N-(3,5-Di-t-butyl-4-hydroxyphenyl)-carbaminsäureoctylester.
   1.12 Ester der β-(3,5-Di-t-butyl-4-hydroxyphenyl)-propionsäure mit ein- oder mehrwertigen Alkoholen, wie mit Methanol, Ethanol, Octadecanol, 1,6-Hexandiol, 1,9-Nonandiol, Ethylenglykol, 1,2-Propandiol, Neopentylglykol, Thiodiethylenglykol, Diethylenglykol, Triethylenglykol, Pentaerythrit, Tris-(hydroxyethyl)-isocyanurat, N,N'-Bis-(hydroxyethyl)-oxalsäurediamid, 3-Thia-undecanol, 3-Thia-pentadecanol, Trimethylhexandiol, Trimethylolpropan, 4-Hydroxymethyl-1-phospha-2,6,7-trioxabicyclo-[2,2,2]-octan.
   1.13 Ester der β-(5-t-Butyl-4-hydroxy-3-methylphenyl)-propionsäure mit ein- oder mehrwertigen Alkoholen, wie beispielsweise mit Methanol, Ethanol, Octadecanol, 1,6-Hexandiol, 1,9-Nonandiol, Ethylenglykol, 1,2-Propandiol, Neopentylglykol, Thiodietyhlenglykol, Diethylenglykol, Triethylenglykol, Pentaerythrit, Tris-(hydroxy)-ethyl-isocyanurat, N,N'-Bis-(hydroxyethyl)-oxalsäurediamid, 3-Thia-undecanol, 3-Thia-pentadecanol, Trimethylhexandiol, Trimethylolpropan, 4-Hydroxymethyl-1-phospha-2,6,7-trioxabicyclo-[2,2,2]-octan.
   1.14 Ester der β-(3,5-Dicyclohexyl-4-hydroxyphenyl)-propionsäure mit ein- oder mehrwertigen Alkoholen, wie beispielsweise Methanol, Ethanol, Octadecanol, 1,6-Hexandiol, 1,9-Nonandiol, Ethylenglykol, 1,2-Propandiol, Neopentylglykol, Thiodiethylenglykol, Diethylenglykol, Triethylenglykol, Pentaerythrit, Tris-(hydroxy)ethyl-isocyanurat, N,N'-Bis-(hydroxyethyl)-oxalsäurediamid, 3-Thiaundecanol, 3-Thia-pentadecanol, Trimethylhexandiol, Trimethylolpropan, 4-Hydroxymethyl-1-phospha-2,6,7-trioxabicyclo-(2,2,2)-octan.
   1.15 Ester der 3,5-Di-t-butyl-4-hydroxyphenylessigsäure mit ein- oder mehrwertigen Alkoholen, wie beispielsweise mit Methanol, Ethanol, Octadecanol, 1,6-Hexandiol, 1,9-Nonandiol, Ethylenglykol, 1,2-Propandiol, Neopentylglykol, Thiodiethylenglykol, Diethylenglykol, Triethylenglykol, Pentaerythrit, Tris-(hydroxy)ethyl-isocyanurat, N,N'-Bis-(hydroxyethyl)-oxalsäurediamid, 3-Thiaundecanol, 3-Thia-pentadecanol, Trimethylhexandiol, Trimethylolpropan, 4-Hydroxymethyl-1-phospha-2,6,7-trioxabicyclo-[2,2,2]-octan.
   1.16 Ester der 3,3-Bis-(3'-t-butyl-4'-hydroxyphenyl)-buttersäure mit ein- oder mehrwertigen Alkoholen, wie beispielsweise mit Methanol, Ethanol, Octadecanol, 1,6-Hexandiol, 1,9-Nonandiol, Ethylenglykol, 1,2-Propandiol, Neopentylglykol, Thiodietyhlenglykol, Diethylenglykol, Triethylenglykol, Pentaerythrit, Tris-(hydroxy)-ethyl-isocyanurat, N,N'-Bis-(hydroxyethyl)-oxalsäurediamid, 3-Thia-undecanol, 3-Thia-pentadecanol, Trimethylhexandiol, Trimethylolpropan, 4-Hydroxymethyl-1-phospha-2,6,7-trioxabicyclo-[2,2,2]-octan.
   1.17 Amide der β-(3,5-Di-t-butyl-4-hydroxyphenyl)-propionsäure, wie beispielsweise N,N'-Bis-(3,5-di-t-butyl-4-hydroxyphenylpropionyl)-hexamethylendiamin, N, N' -Bis-(3, 5-di-t-butyl-4-hydroxyphenylpropionyl)-trimethylendiamin, N,N'-Bis-(3,5-di-t-butyl-4-hydroxyphenylpropionyl)-hydrazin.
2. UV-Absorber und Lichtschutzmittel
   2.1 2-(2'-Hydroxyphenyl)-benztriazole, wie beispielsweise 2-(2'-Hydroxy-5'-methylphenyl)-benztriazol, 2-(3',5'-Di-t-butyl-2'-hydroxyphenyl)-benztriazol, 2-(5'-t-Butyl-2'-hydroxyphenyl)-benztriazol, 2-[2'-Hydroxy-5'-(1,1,3,3-tetramethylbutyl)phenyl]-benztriazol, 2-(3',5'-Di-t-butyl-2'-hydroxyphenyl)-5-chlor-benztriazol, 2-(3'-t-Butyl-2'-hydroxy-5'-methylphenyl)-5-chlor-benztriazol, 2-(3'-sec-Butyl-5'-t-butyl-2'-hydroxyphenyl)-benztriazol, 2-(2'-Hydroxy-4'-octoxyphenyl)-benztriazol, 2-(3',5'-Di-t-amyl-2'-hydroxyphenyl)-benztriazol, 2-(3',5'-Bis-(α,α-dimethylbenzyl)-2'-hydroxyphenyl)-benztriazol, Mischung aus 2-(3'-t-Butyl-2'-hydroxy-5'-(2-octyloxycarbonylethyl)-phenyl)-5-chlor-benztriazol, 2-(3'-t-Butyl-5'-[2-(2-ethylhexyloxy)-carbonylethyl]-2'-hydroxyphenyl)-5-chlor-benztriazol, 2-(3'-t-Butyl-2'-hydroxy-5'-(2-methoxycarbonylethyl)phenyl)-5-chlor-benztriazol, 2-(3'-t-Butyl-2'-hydroxy-5'-(2-methoxycarbonylethyl)phenyl)-benztriazol, 2-(3'-t-Butyl-2'-hydroxy-5'-(2-octyloxycarbonylethyl)phenyl)-benztriazol, 2-(3'-t-Butyl-5'-[2-(2-ethylhexyloxy)-carbonylethyl]-2'-hydroxyphenyl)-benztriazol, 2-(3'-Dodecyl-2'-hydroxy-5'-methylphenyl)-benztriazol, und 2-(3'-t-Butyl-2'-hydroxy-5'-(2-isooctylocycarbonylethyl)phenyl-benztriazol, 2,2'-Methylen-bis-[4-(1,1,3,3-tetramethylbutyl)-6-benztriazol-2-yl-phenol]; Umesterungsprodukt von 2-[3'-t-Butyl-5'(2-methoxycarbonylethyl)-2'-hydroxy-phenyl]-benztriazol mit Polyethylenglykol 300.
   2.2 2-Hydroxybenzophenone, wie beispielsweise das 4-Hydroxy-, 4-Methoxy-, 4-Octoxy, 4-Decyloxy, 4-Dodecyloxy-, 4-Benzyloxy-, 4,2',4'-Trihydroxy-, 2'-Hydroxy-4,4'-dimethoxy-Derivat.
   2.3 Ester von gegebenenfalls substituierten Benzoesäuren, wie beispielsweise 4-t-Butyl-phenylsalicylat, Phenylsalicylat, Octylphenyl-salicylat, Dibenzoylresorcin, Bis-(4-t-butylbenzoyl)-resorcin, Benzoylresorcin, 3, 5-Di-t-butyl-4-hydroxybenzoesäure-2,4-di-t-butylphenylester, 3, 5-Di-t-butyl-4-hydroxybenzosäure-hexadecylester, 3,5-Di-t-butyl-4-hydroxybenzoesäure-octadecylester, 3,5-Di-t-butyl-4-hydroxybenzoesäure-2-methyl-4,6-di-t-butylphenylester.
   2.4 Acrylate wie beispielsweise α-Cyan-β,β-diphenylacrylsäure-ethylester oder -isooctylester, α-Carbomethoxy-zimtsäuremethylester, α-Cyano-β-methyl-p-methoxyzimtsäuremethylester oder -butylester, α-Carbomethoxy-p-methoxy-zimtsäuremethylester, N-(β-Carbomethoxy-β-cyanovinyl)-2-methyl-indolin.
   2.5 Nickelverbindungen wie beispielsweise Nickelkomplexe des 2,2'-Thio-bis-[4-(1,1,3,3 tetramethylbutyl)-phenols] wie der 1:1 oder der 1:2 Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-t-butyl-benzylphosphonsäure-monoalkylestern, wie vom Methyl- oder Ethylester, Nickelkomplexe von Ketoximen, wie von 2-Hydroxy-4-methyl-phenylundecylketoxim, Nickelkomplexe des 1-Phenyl-4-lauroyl-5-hydroxy-pyrazols, gegebenenfalls mit zusätzlichen Liganden.
   2.6 Sterisch gehinderte Amine, wie beispielsweise Bis-(2,2,6,6-tetramethylpiperidyl)-sebacat, Bis-(2,2,6,6-tetramethyl-piperidyl)-glutarat, Bis-(2,2,6,6-tetramethyl-piperidyl)-succinat, Bis-(1,2,2,6,6-pentamethylpiperidyl)-sebacat, Bis-(1,2,2,6,6-pentamethylpiperidyl)-glutarat, Bis-(1,2,2,6,6-pentamethylpiperidyl)-sebacat, n-Butyl-3,5-di-t-butyl-4-hydroxybenzyl-malonsäure-bis(1,2,2,6,6-pentamethylpiperidyl)-ester, 2,2,6,6-Tetramethylpiperidylbehenat, 1,2,2,6,6-Pentamethylpiperidylbehenat, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-tetramethyl-4-piperidyl)-hexamethylendiamin und 4-t-Octylamino-2,6-dichlor-1,3, 5-s-triazin, Tris-(2,2, 6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3, 5, 5-tetramethyl-piperazinon), 4-Benzoyl-2,2, 6, 6-tetramethylpiperidin, 4-Stearyloxy-2,2,6,6-tetramethylpiperidin, 4-Stearoyloxy-2,2,6,6-tetramethylpiperidin, 4-Stearyloxy-1 ,2,2,6,6-pentamethylpiperidin, 4-Stearoyloxy-1,2,2,6,6-pentamethylpiperidin, Bis-(1,2,2,6,6-pentamethylpiperidyl)-2-n-butyl-2-(2-hydroxy-3,5-di-t-butylbenzyl)-malonat, Bis-(1 ,2,2,6,6-pentamethylpiperidyl)-2-n-butyl-2-(4-hydroxy-3,5-di-t-butylbenzyl)-malonat, 3-n-Octyl-7,7,9,9-tetramethyl-1,3,8-triazaspiro [4,5] decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-methoxypropylamino-2,2,6,6-tetramethylpiperidyl)-1,3,5-triazin und 1,2-Bis-(3-aminopropylamino)äthan, Kondensationsprodukt aus 2-Chlor-4,6-di-(4-methoxypropylamino-1,2,2,6,6-pentamethylpiperidyl)-1,3,5-triazin und 1,2-Bis-(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-ethan, Umsetzungsprodukte von 2-Chlor-4,6-di-(4-n-butylamino-2,2,6,6-tetramethylpiperidyl)-1,3,5-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)äthan, Umsetzungsprodukte von 2-Chlor-4,6-di-(4-n-butylamino-1,2,2,6, 6-pentamethylpiperidyl)-1,3,5-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)äthan, Umsetzungsprodukte von 2-Chlor-4,6-di-(4-n-butylamino-2,2,6,6-tetramethylpiperidyl)-1,3,5-triazin und 4-t-Octylamino-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-1,2,2,6,6-pentamethylpiperidyl)-1,3,5-triazin und 4-t-Octylamino-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-2,2,6,6-tetramethylpiperidyl)-1,3,5-triazin und 4-(4-n-Butylamino-2,2,6,6-tetramethylpiperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, Umsetzungsprodukte von 2-Chlor-4,6-di-(4-n-butylamino-1,2,2,6,6-pentamethylpiperidyl)-1,3,5-triazin und 4-(4-n-Butylamino-2,2,6,6-tetramethylpiperidyl)-2,6-dichlor-1,3,5-s-triazin mit ein- oder mehrwertigen Aminen, wobei zwischen einem und allen aktiven H-Atomen am Amin ersetzt werden, wie mit Ethylendiamin, Diethylentriamin, Triethylentetramin, Hexamethylendiamin, 1,2-Bis-(3-aminopropylamino)ethan, 3-Dodecyl-7,7,9,9-tetramethyl-1,3,8-triazaspiro-[4,5]-decan-2,4-dion, Oligomerisiertes 2,2,4,4-Tetramethyl-20-(oxiranylmethyl)-7-oxa-3,20-diaza-dispiro-[5.1.11.2]-heneicosan-21-on, Oligomerisiertes 1,2,2,4,4-Pentamethyl-20-(oxiranylmethyl)-7-oxa-3, 20-diaza-dispiro-[5.1.11.2]-heneicosan-21-on, Oligomerisiertes 1-Acetyl-2,2,4,4-tetramethyl-20-(oxiranylmethyl)-7-oxa-3,20-diaza-dispiro-[5.1.11.2]-heneicosan-21-on, Dodecyl-1-(2, 2,4,4-tetramethyl-4-piperidyl)pyrrolidin-2, 5-dion, 3-Dodecyl-1-(1,2,2,6,6-pentamethyl-4-piperidyl)-pyrrolidin-2,5-dion, 2,2,4,4-Tetramethyl-7-oxa-3,20-diaza-dispiro-[5.1.11.2]-heneicosan-21-on, 2,2,4,4-Tetramethyl-7-oxa-21-oxo-3,20-diaza-dispiro-[5.1.11.2]-heneicosan-3-propansäure-dodecylester, 2,2,4,4-Tetramethyl-7-oxa-21-oxo-3,20-diaza-dispiro-[5.1.11.2]-heneicosan-3-propansäure-tetradecylester, 2, 2, 3,4,4-Pentamethyl-7-oxa-3,20-diaza-dispiro-[5.1.11.2]-heneicosan-21-on, 2, 2,3,4,4-Pentamethyl-7-oxa-21-oxo-3, 20-diaza-dispiro-[5.1.11.2]-heneicosan-3-propansäure-dodecylester, 2,2,3,4,4-Pentamethyl-7-oxa-21-oxo-3,20-diaza-dispiro-[5.1.11.2]-heneicosan-3-propansäure-tetradecylester, 3-Acetyl-2,2,4,4-tetramethyl-7-oxa-3,20-diaza-dispiro-[5.1.11.2]-heneicosan-21-on, 3-Acetyl-2,2,4,4-tetramethyl-7-oxa-21-oxo-3,20-diaza-dispiro-[5.1.11.2]-heneicosan-3-propansäuredodecylester, 3-Acetyl-2,2,4,4-tetramethyl-7-oxa-21-oxo-3,20-diaza-dispiro-[5.1.11.2]-heneicosan-3-propansäure-tetradecylester,1,1',3,3',5,5'-Hexahydro-2,2',4,4',6,6'-hexaaza-2,2',6,6'-bismethano-7,8-dioxo-4,4'-bis-(1,2,2,6,6-pentamethyl-4-piperidyl)-biphenyl, Poly-N,N'-bis-(2,2,6,6-tetramethyl-4-piperidyl)-1,8-diazadecyclen, Additionsverbindung von 2,2,6,6-Tetramethyl-4-allyloxypiperidin und Polymethylhydrogensiloxan (Molmasse bis 4000), Additionsverbindung von 1,2,2,6,6-Pentamethyl-4-allyloxy-piperidin und Polymethylhydrogensiloxan (Molmasse bis 4000), N,N'-Di-formyl-N,N'-bis-(2,2,6,6-tetramethyl-4-piperidinyl)-hexamethylendiamin, N,N'-Di-formyl-N,N'-bis-(1,2,2,6,6-pentamethyl-4-piperidinyl)-hexamethylendiamin, 5,11-Bis-(2,2,6,6-tetramethyl-4-piperidinyl)-3,5,7,9,11,13-hexaaza-tetracyclo-[7.4.0.0^{2,7}.1^{3,13}]-tetradecan-8,14-dion, 5,11-Bis-(1,2,2,6,6-pentamethyl-4-piperidinyl)-3,5,7,9,11,13-hexaazatetracyclo-[7.4.0.0^{2.7}.1^{3,13}]-tetradecan-8,14-dion, 7,7,9,9-Tetramethyl-8-acetyl-3-dodecyl-1,3,8-triaza-spiro-[4.5]-decan-2,4-dion, [(4-Methoxyphenyl)-methylen]-propandisäure-bis-(2,2,6,6-tetramethyl-4-piperidinyl)-ester, [(4-Methoxyphenyl)-methylen]-propandisäure-bis-(1,2,2,6,6-pentamethyl-4-piperidinyl)-ester, 2,4,6-Tris-(N-cyclohexyl-N-[2-(3,3,4,5,5-pentamethyl-piperazinon-1 -yl)-ethyl]-amino)-1,3,5-triazin, Copolymerisat aus Styrol mit α-Methylstyrol und Maleinsäureanhydrid umgesetzt mit 4-Amino-2,2,6,6-tetramethylpiperidin und Octadecylamin, Copolymerisat aus Styrol mit α-Methylstyrol und Maleinsäureanhydrid umgesetzt mit 4-Amino-1,2,2,6,6-pentamethylpiperidin und Octadecylamin, Polycarbonat mit 2,2'-[(2,2,6,6-Tetramethyl-4-piperidinyl)-imino]-bis-[ethanol] als Diolkomponente, Polycarbonat aus 2,2'-[(1,2,2,6,6-Pentamethyl-4-piperidinyl)-imino]-bis-[ethanol] als Diolkomponente, Copolymer aus Maleinsäureanhydrid und einem α-Olefin bis C₃₀ umgesetzt mit 4-Amino-2,2,6,6-tetramethylpiperidin, Copolymer aus Maleinsäureanhydrid und einem α-Olefin bis C₃₀ umgesetzt mit 1-Acetyl-4-amino-2,2,6,6-tetramethylpiperidin, Copolymer aus Maleinsäureanhydrid und einem α-Olefin bis C₃₀ umgesetzt mit 4-Amino-1,2,2,6,6-pentamethylpiperidin, sowie die N-Alkyl- und N-Aryloxyderivate der oben genannten Verbindungen mit freien NH-Gruppen am Piperidin, speziell α-Methylbenzyloxy- und Alkyloxy- von C₁ bis C₁₈.
   2.7 Oxalsäurediamide, wie beispielsweise 4,4'-Di-octyloxy-oxanilid, 2,2'-Di-octyloxy-5,5'-di-t-butyl-oxanilid, 2,2'-Di-dodecyloxy-5,5'-di-t-butyl-oxanilid, 2-Ethoxy-2'-ethyl-oxanilid, N,N'-Bis-(3-dimethylaminopropyl)-oxalamid, 2-Ethoxy-5-t-butyl-2'-ethyloxanilid und dessen Gemisch mit 2-Ethoxy-2'-ethyl-5,4'-di-t-butyloxanilid, Gemische von o- und p-Methoxy- sowie von o- und p-Ethoxy-di-substituierten Oxaniliden.
   2.8 2-(2-Hydroxyphenyl)-1,3,5-triazine, wie beispielsweise 2,4,6-Tris-(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis-(2,4-dimethylphenyl)-1,3,5-triazin, 2-(2,4-Dihydroxyphenyl)-4,6-bis-(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-13,5-triazin, 2-(2-Hydroxy-4-dodecyloxyphenyl)-4,6-bis(2,4-di-methylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.
3. Metalldesaktivatoren, wie beispielsweise N,N'-Diphenyloxalsäurediamid, N-Salicylal-N'-salicyloylhydrazin, N,N'-Bis-(salicyloyl)-hydrazin, N,N'-Bis-(3,5-di-t-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-bis-salicyloyloxalsäure-dihydrazid, N,N'-Bis-salicyloyl-thiopropionsäure-dihydrazid.
4. Phosphite und Phosphonite, wie beispielsweise Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-t-butylphenyl)-phosphit, Diisodecylpentaerythrit-diphosphit, Bis-(2,4-di-t-butylphenyl)-pentaerythritdiphosphit, Bis-(2,6,-di-t-butyl-4-methylphenyl)-pentaerythritdiphosphit, Bis-isodecyloxy-pentaerythritdiphosphit, Bis-(2,4-di-t-butyl-6-methylphenyl)-pentaerythritdiphosphit, Bis-(2,4,6-tri-t-butylphenyl)-pentaerytritdiphosphit, Tristearyl-sorbit-triphosphit, Tetrakis-(2,4-di-t-butylphenyl)-4,4'-biphenylen-diphosphonit, 6-lsooctyloxy-2,4,8,10-tetra-t-butyl-12H-dibenz[d,g]-1,3,2-dioxaphosphocin, 6-Fluor-2,4,8,10-tetra-t-butyl-12-methyl-dibenz-[d,g]-1,3,2-dioxaphophocin, Bis-(2,4-di-t-butyl-6-methylphenyl)-methylphosphit, Bis-(2,4-di-t-butyl-6-methylphenyl)-ethylphosphit, Tris(2-t-butyl-4-thio- (2'-methenyl-4'-hydroxy-5'-t-butyl)-phenyl-5-methenyl)-phenylphosphit.
5. Peroxidzerstörende Verbindungen wie beispielsweise Ester der β-Thio-dipropionsäure, beispielsweise der Lauryl-, Stearyl-, Myristyl- oder Tridecylester, Mecaptobenzimidazol, das Zinksalz des 2-Mercaptobenzimidazols, Zink-alkyl-dithiocarbamate, Zinkdibutyl-dithiocarbamat, Dioctadecylmonosulfid, Dioctadecyldisulfid, Pentaerytrit-tetrakis-(β-dodecylmercapto)-propionat.
6. Polyamidstabilisatoren, wie beispielsweise Kupfersalze in Kombination mit Jodiden und/oder Phosphorverbindungen und Salze des zweiwertigen Mangans.
7. Basische Co-Stabilisatoren wie beispielsweise Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie beispielsweise 4-t-Butylbenzoesäure, Adipinsäure, Diphenylessigsäure.
9. Füllstoffe und Verstärkungsmittel, wie beispielsweise Calciumcarbonat, Silikate, Glasfasern, Asbest, Talk, Kaolin, Glimmer, Bariumsulfat, Metalloxide und - hydroxide, Ruß, Graphit.
10. Sonstige Zusätze, wie beispielsweise Weichmacher, Gleitmittel, Emulgatoren, Pigmente, Optische Aufheller, Flammschutzmittel, Antistatika, Treibmittel.

Die Additive werden nach allgemein üblichen Methoden in die organischen Polymeren eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglichem Verdunstenlassen des Lösemittels kann die Einarbeitung erfolgen. Die Verbindungen sind auch wirksam, wenn sie in ein bereits granuliertes Polymer nachträglich in einem gesonderten Verarbeitungsschritt eingebracht werden.

Die erfindungsgemäß zu verwendenden Verbindungen können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

Die erfindungsgemäßen Verbindungen sind hervorragende Lichtstabilisatoren, die sich durch eine geringe Flüchtigkeit auszeichnen.

Die nachfolgenden Beispiele sollen die Erfindung näher erläutern.

### Beispiel 1

15,7 g 20,20'-(2-Hydroxy-1,3-propandiyl)-bis-[2,2,4,4-tetramethyl-7-oxa-3,20-diazadispiro-[5.1.11.2]-heneicosan-21-on] wurden zusammen mit 2,2 g Triethylamin in 80 cm³ Xylol vorgelegt. Dazu wurden 2,8 g 2-Chlor-1,3,2-dioxaphospholan bei Raumtemperatur zugegeben und das Gemisch anschließend 5 Stunden auf Rückflußtemperatur erhitzt. Danach versetzte man den Ansatz mit Wasser, stellte die wässrige Phase alkalisch und schüttelte sie mehrmals mit Xylol aus. Nach dem Trocknen mit Magnesiumsulfat und Einengen der Lösung wurde das zurückbleibende Harz aus Ethanol umkristallisiert. 11,4 g eines weißen Produktes mit einem Schmelzpunkt von 128°C wurden erhalten.

### Beispiel 2

16,2 g 20,20'-(2-Hydroxy-1,3-propandiyl)-bis-[2,2,3,4,4-pentamethyl-7-oxa-3,20-diazadispiro-[5.1.11.2]-heneicosan-21-on] und 2,2 g Triethylamin wurden in 80 cm³ Toluol vorgelegt. Bei Raumtemperatur wurde langsam eine Lösung von 2,8 g 2-Chlor-1,3,2-dioxaphospholan in 20 cm³ Toluol unter Rühren zugetropft; anschließend wurde der Ansatz 5 Stunden auf Rückflußtemperatur erhitzt. Zur Aufarbeitung goß man das Gemisch auf Wasser, trennte die Phasen, extrahierte die wässrige Phase zweimal mit Essigsäureethylester und trocknete die vereinigten organischen Phasen. Nach dem Einengen wurden 16,2 g der eines schwach gelben, nicht kristallisierenden Harzes erhalten.

### Beispiel 3

39,2 g 20,20'-(2-Hydroxy-1,3-propandiyl)-bis-[2,2,4,4-tetramethyl-7-oxa-3,20-diazadispiro-[5.1.11.2]-heneicosan-21-on] und 5,1 g Triethylamin wurden in 100 cm³ Toluol vorgelegt. Bei Raumtemperatur wurden langsam 5,4 g Chlortrimethylsilan zugetropft und anschließend das Gemisch 8 h auf 50°C erhitzt. Der Ansatz wurde mit Wasser ausgeschüttelt und die organische Phase mit Natriumsulfat getrocknet. Beim Abkühlen kristallisierten 23,4 g Produkt als weißer Feststoff mit einem Schmelzpunkt von 187°C aus.

### Beispiel 4

0,5 g Natriumhydrid wurden in 50 cm³ Methyl-t-butyl-ether vorgelegt und eine Lösung von 16,2 g 20,20'-(2-Hydroxy-1,3-propandiyl)-bis-[2,2,3,4,4-pentamethyl-7-oxa-3,20-diazadispiro-[5.1.11.2]-heneicosan-21-on] in 100 cm³ Methyl-t-butyl-ether bei Raumtemperatur langsam zugetropft. Nach 1 Stunde wurden 2,4 g Trimethylchlorsilan in 50 cm³ Methyl-t-butyl-ether ebenfalls bei Raumtemperatur zugetropft und der Ansatz 20 Stunden gerührt. Zuletzt erhitzte man das Gemisch für 3 Stunden unter Rückfluß. Überschüssiges Natriumhydrid wurde mit wenig Methanol zerstört und der Ansatz auf Wasser gegossen. Die organische Phase wurde abgetrennt, mit Wasser ausgeschüttelt und mit Natriumsulfat getrocknet. Nach dem Einengen der Lösung blieben 13,5 g der gesuchten Verbindung als farblose, glasartige Masse zurück.

### Beispiel 5

Zu einer Lösung von 5,1 g Triethylamin und 39,2 g 20,20'-(2-Hydroxy-1,3-propandiyl)-bis-[2,2,4,4-tetramethyl-7-oxa-3,20-diazadispiro-[5.1.11.2]-heneicosan-21-on] in Xylol wurden 3,2 g Dichlordimethylsilan zugetropft. Anschließend erhitzte man das Gemisch 12 h auf 50°C. Der Ansatz wurde mit Wasser ausgeschüttelt, die organische Phase getrocknet und eingeengt. Das zurückbleibende Öl ließ sich aus Aceton umkristallisieren, wobei 41,1 g eines weißen Feststoffes mit einem Schmelzpunkt von 161°C erhalten wurden.

### Beispiel 6

Zu einer Suspension von 0,5 g Natiumhydrid in 50 cm³ Methyl-t-butyl-ether wurde eine Lösung von 16,2 g 20,20'-(2-Hydroxy-1,3-propandiyl)-bis-[2,2,3,4,4-pentamethyl-7-oxa-3,20-diazadispiro-[5.1.11.2]-heneicosan-21-on] in 100 cm³ Methyl-t-butyl-ether zugetropft und das Gemisch 1 h gerührt. Anschließend tropfte man 1,3 g Dichlordimethylsilan zu und rührte weitere 12 h unter Rückfluß. Restliches Natriumhydrid wurde danach mit wenig Methanol zerstört und der Ansatz mehrmals mit Wasser ausgeschüttelt. Nach dem Trocknen und Einengen der organischen Phase blieben 13,3 g der gesuchten Verbindung als leicht gelbe, harzartige Masse zurück.

### Beispiel 7

Zu 15,7 g 20,20'-(2-Hydroxy-1,3-propandiyl)-bis-[2,2,4,4-tetramethyl-7-oxa-3,20-diazadispiro-[5.1.11.2]-heneicosan-21-on] und 2,2 g Triethylamin in 80 cm³ Xylol wurden 2,2 g Chlorameisensäuremethylester gegeben und das Gemisch danach 5 Stunden unter Rückfluß erhitzt. Der Ansatz wurde dann mehrmals mit Wasser ausgeschüttelt und die organische Phase getrocknet und eingeengt. Das zurückbleibende Harz ließ sich aus Aceton umkristallisieren, so daß 13,6 g farblose Kristalle erhalten wurden.

### Beispiel 8

14 cm³ einer 1,6-molaren Butyllithium-Lösung, verdünnt mit 20 cm³ Toluol, wurden bei Raumtemperatur zu einer Lösung von 16,2 g 20,20'-(2-Hydroxy-1,3-propandiyl)-bis-[2,2,3,4,4-pentamethyl-7-oxa-3,20-diazadispiro-[5.1.11.2]-heneicosan-21-on] in 60 cm³ Toluol zugetropft. Nach 1 Stunde wurde eine Lösung von 2,1 g Chlorameisensäuremethylester in 20 cm³ Toluol zugetropft und der Ansatz 20 Stunden bei Raumtemperatur gerührt. Zur Aufarbeitung wurde das Gemisch auf Wasser gegossen, die organische Phase abgetrennt und mit Natriumsulfat getrocknet. Nach dem Einengen der Lösung wurden 14,2 g der gesuchten Verbindung als farblose, glasartige Masse erhalten.

### Beispiel 9

16,2 g 20,20'-(2-Hydroxy-1,3-propandiyl)-bis-[2,2,3,4,4-pentamethyl-7-oxa-3,20-diazadispiro-[5.1.11.2]-heneicosan-21-on] und 2,8 g Kaliumkarbonat wurden in 80 cm³ Toluol vorgelegt. Hierzu gab man 9,1 g 2-Chloro-4,6-bis-[1,2,2,6,6-pentamethyl-4-piperidinyloxy]-1,3,5-triazin und erhitzte das Gemisch 5 Stunden unter Rückfluß. Die abgekühlte Mischung wurde mit 100 cm³ Toluol verdünnt und viermal mit Wasser ausgeschüttelt. Nach dem Trocknen und Abdestillieren des Lösemittels blieben 21,2 g einer farblosen, glasartigen Masse zurück.

### Beispiel 10

Die Flüchtigkeiten von erfindungsgemäßen Verbindungen wurden in einer Apparatur zur thermogravimetrischen Analyse bestimmt. Gleiche Mengen (500 mg) der erfindungsgemäßen Verbindungen wurden dazu in Stickstoffatomosphäre mit einer Aufheizgeschwindigkeit von 2 K/min bis auf 300°C erhitzt und der Substanzverlust in mg/cm² Probenoberfläche gemessen. Die Ergebnisse zeigt nachstehende Tabelle:

| Gewichtsverlust in mg/cm² beim Erreichen von ... °C | | | | |
|---|---|---|---|---|
| Verbindung gem. Beispiel | 220 | 260 | 300 | 10 min bei 300 °C |
| 3 | 0.0 | 0.2 | 0.8 | 2.0 |
| 4 | 0.3 | 0.3 | 0.7 | 1.2 |
| 5 | 1.7 | 2.0 | 2.3 | 2.7 |
| 8 | 0.2 | 1.9 | 3.2 | 3.8 |
| | | | | |
| Vgl.¹⁾: 14 | 0.6 | 2.5 | 7.7 | 12.6 |
| Vgl.¹⁾: 15 | 1.4 | 3.9 | 8.1 | 13.3 |

| | | | | |
|---|---|---|---|---|
| Vgl.¹⁾: aus EP 57 885 | | | | |

## Patentansprüche

1. Polyalkylpiperidin-Verbindungen der Formel I 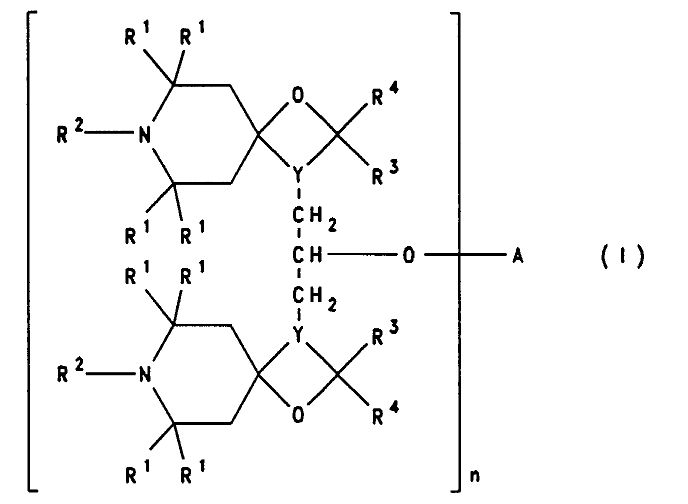 worin
Y eine der Gruppen 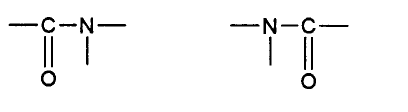 ist,
R¹ ein Wasserstoffatom oder eine Alkylgruppe bedeutet,
R² ein Wasserstoffatom oder eine Alkylgruppe ist,
R³ und R⁴ gleich oder verschieden sind und ein Wasserstoffatom oder eine Alkylgruppe bedeuten, oder R³ und R⁴ zusammen mit dem sie verbindenden C-Atom einen Ring mit 5 bis 12 Ringliedern bilden,
n = 1, 2 oder 3 bedeutet und
A bei n = 1
-COOR⁵, -P(OR⁶)(OR⁷), -Si(OR⁵)(OR⁵)(OR⁵), -SiR⁵R⁵R⁵ oder die Gruppe 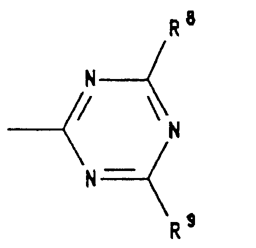
A bei n = 2
-CO-, =POR⁵, =Si(OR⁵)(OR⁵), =SiR⁵R⁵ oder die Gruppe 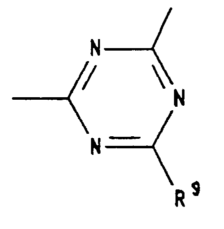
A bei n = 3
≡Si(OR⁵), ≡SiR⁵ oder die Gruppe 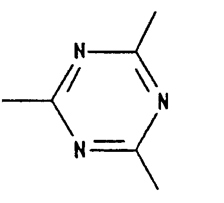 bedeutet,
R⁵ eine C₁ - C₁₈ -Alkylgruppe, eine C₂ - C₁₈ -Alkenylgruppe, eine C₂ - C₁₈ - Alkinylgruppe, eine C₅ - C₁₂ -Cycloalkylgruppe, eine C₆ - C₁₀ -Bicycloalkylgruppe, eine C₅ - C₈ -Cycloalkenylgruppe, eine C₆ - C₁₀-Arylgruppe, eine C₇ - C₉ - Aralkylgruppe, eine C₇ - C₉ -Alkarylgruppe, gegebenenfalls substituiert durch C₁ - C₄ -Alkyl oder Phenyl, bedeutet, wobei mehrere Reste R⁵ gleich oder verschieden sein können,
R⁶ und R⁷ gleich oder verschieden sind und eine C₁ - C₁₈ -Alkylgruppe, eine C₂ - C₁₈ -Alkenylgruppe, eine C₂ - C₁₈ -Alkinylgruppe, eine C₅ - C₁₂ -Cycloalkylgruppe, eine C₆ - C₁₀ -Bicycloalkylgruppe, eine C₅ - C₈ -Cycloalkenylgruppe, eine C₆ - C₁₀ - Arylgruppe, eine C₇ - C₉ -Aralkylgruppe oder eine C₇ - C₉ -Aralkylgruppe, substituiert durch C₁ - C₄ -Alkyl oder Phenyl, bedeuten, oder ein Rest sind der Formel II oder III 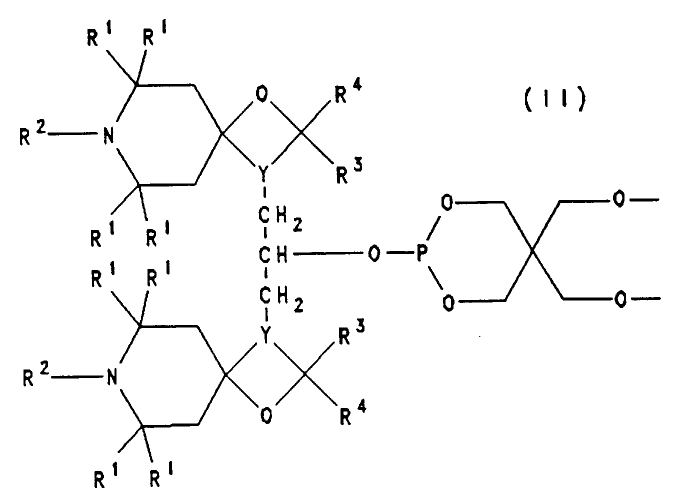 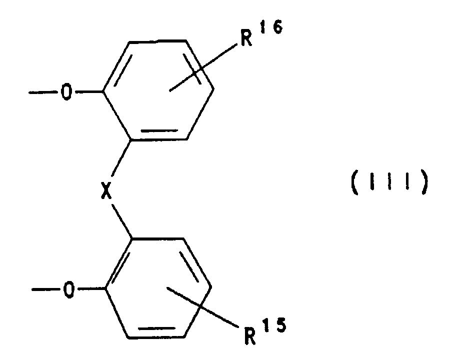 worin R¹, R², R³, R⁴ und R⁵ die obengenannte Bedeutung haben,
und X eine direkte Bindung oder Methylengruppe oder eine 1,1-Alkylidengruppe mit 2 bis 5 C-Atomen bedeutet,
R⁸ und R⁹ gleich oder verschieden sind und -OR⁵, NHR⁵, -NR¹¹R¹², oder eine der beiden Gruppen sind der Formeln 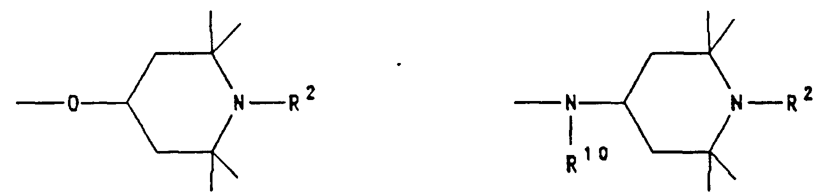 worin R¹⁰ ein Wasserstoffatom, eine C₁ - C₁₂ -Alkylgruppe, eine C₅ - C₇ - Cycloalkylgruppe, eine C₇ - C₉ -Aralkylgruppe, eine C₈ - C₁₈ -Alkanoylgruppe, eine C₃ - C₅ -Alkenoylgruppe oder eine Benzoylgruppe bedeutet, oder 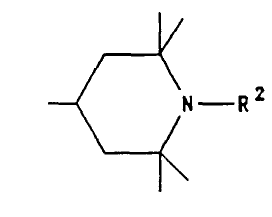 ist,
R¹¹ und R¹² gleich oder verschieden sind und eine C₁ - C₁₀ -Alkylgruppe, eine C₅ - C₇ -Cycloalkylgruppe, eine C₆ - C₁₀ -Arylgruppe bedeuten, oder R¹¹ und R¹² zusammen mit dem Stickstoff einen 5- bis 12-gliedrigen Ring bilden, der auch noch Sauerstoff enthalten kann.

2. Verbindungen gemäß Anspruch 1, dadurch gekennzeichnet, daß in Formel I R¹ eine Methylgruppe ist.

3. Verbindungen gemäß Anspruch 1, dadurch gekennzeichnet, daß in Formel I R² eine Methylgruppe ist.

## Claims

1. A polyalkylpiperidine compound of the formula I 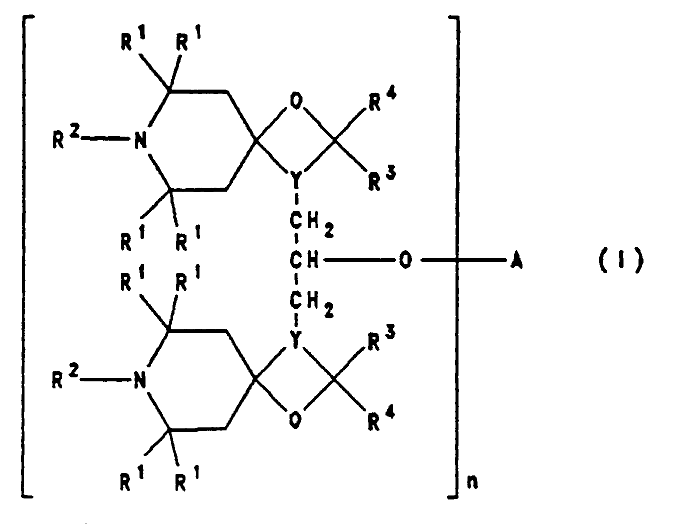 in which
Y is one of the groups 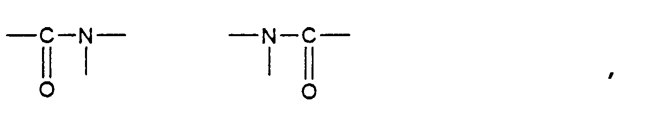
R¹ is a hydrogen atom or an alkyl group,
R² is a hydrogen atom or an alkyl group,
R³ and R⁴ are identical or different and are a hydrogen atom or an alkyl group, or R³ and R⁴, together with the carbon atom connecting them, form a ring having 5 to 12 ring members,
n is 1, 2 or 3, and
A, in the case where n = 1,
is -COOR⁵, -P(OR⁶)(OR⁷), -Si(OR⁵)(OR⁵)(OR⁵), -SiR⁵R⁵R⁵ or the 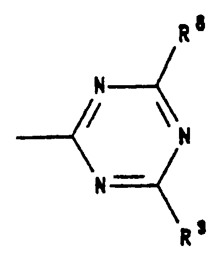 group,
A, in the case where n = 2,
is -CO-, =POR⁵, =Si(OR⁵)(OR⁵), =SiR⁵R⁵ or the 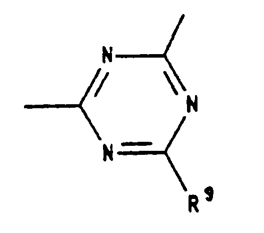 group,
A, in the case where n = 3,
is ≡Si(OR⁵), ≡SiR⁵ or the 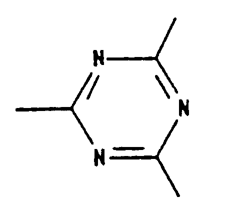 group,
R⁵ is a C₁-C₁₈-alkyl group, a C₂-C₁₈-alkenyl group, a C₂-C₁₈-alkynyl group, a C₅-C₁₂-cycloalkyl group, a C₆-C₁₀-bicycloalkyl group, a C₅-C₈-cycloalkenyl group, a C₆-C₁₀-aryl group, a C₇-C₉-aralkyl group, a C₇-C₉-alkaryl group, unsubstituted or substituted by C₁-C₄-alkyl or phenyl, where a plurality of radicals R⁵ may be identical or different,
R⁶ and R⁷ are identical or different and are a C₁-C₁₈-alkyl group, a C₂-C₁₈-alkenyl group, a C₂-C₁₈-alkynyl group, a C₅-C₁₂-cycloalkyl group, a C₆-C₁₀-bi-cycloalkyl group, a C₅-C₈-cycloalkenyl group, a C₆-C₁₀-aryl group, a C₇-C₉-aralkyl group or a C₇-C₉-aralkyl group, substituted by C₁-C₄-alkyl or phenyl, or are a radical of the formula II or III 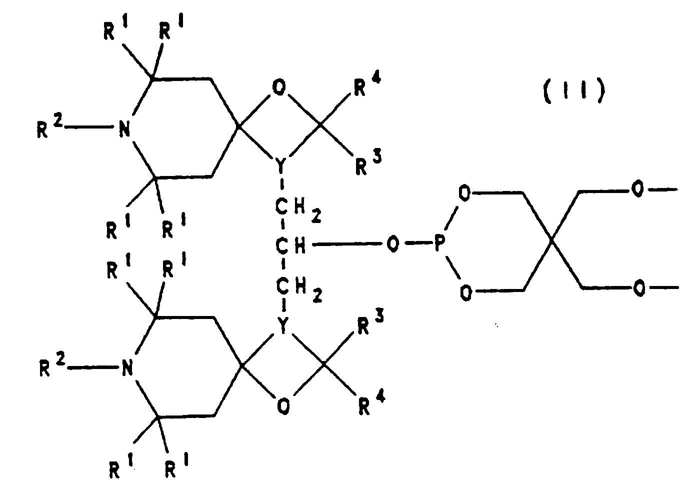 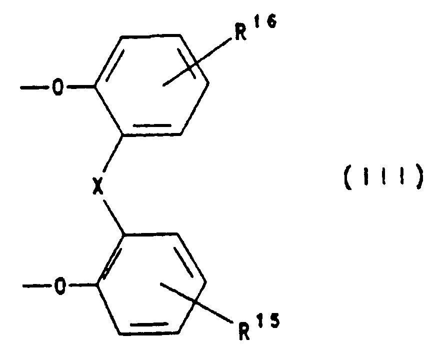 in which R¹, R², R³, R⁴ and R⁵ are as defined above, and X is a direct bond or methylene group or a 1,1-alkylidene group having 2 to 5 carbon atoms,
R⁸ and R⁹ are identical or different and are -OR⁵, -NHR⁵, -NR¹¹R¹² or one of the two groups of the formulae 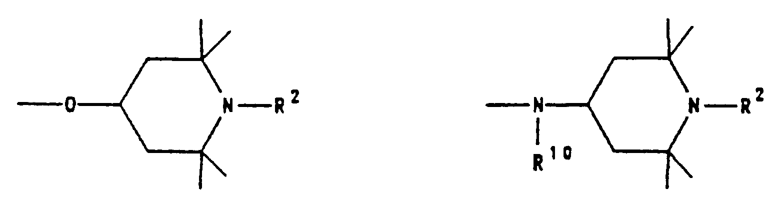 in which R¹⁰ is a hydrogen atom, a C₁-C₁₂-alkyl group, a C₅-C₇-cycloalkyl group, a C₇-C₉-aralkyl group, a C₈-C₁₈-alkanoyl group, a C₃-C₅-alkenoyl group or a benzoyl group, or is 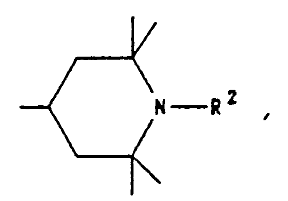 R¹¹ and R¹² are identical or different and are a C₁-C₁₀-alkyl group, a C₅-C₇-cycloalkyl group or a C₆-C₁₀-aryl group, or R¹¹ and R¹², together with the nitrogen, form a 5- to 12-membered ring, which may also contain oxygen.

2. A compound as claimed in claim 1, wherein R¹ in formula I is a methyl group.

3. A compound as claimed in claim 1, wherein R² in formula I is a methyl group.

## Revendications

1. Composés de polyalkylpipéridine de formule I 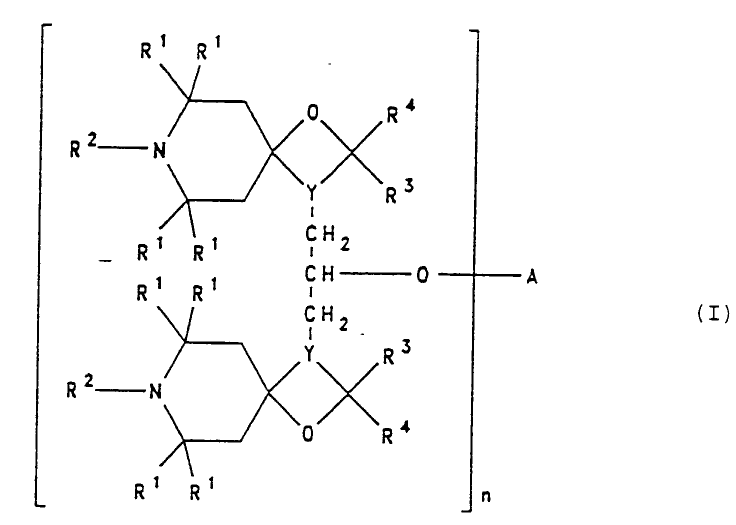 dans laquelle
Y représente un des groupes 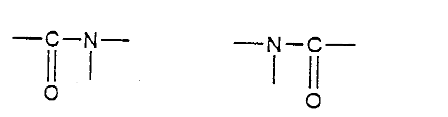
R¹ représente un atome d'hydrogène ou un groupe alkyle,
R² représente un atome d'hydrogène ou un groupe alkyle,
R³ et R⁴ sont identiques ou différents à représentent un atome d'hydrogène ou un groupe alkyle, ou R³ et R⁴ ensemble avec l'atome de carbone les reliant, forment un cycle avec 5 à 12 chaînons,
n = 1, 2 ou 3, et
A, lorsque n = 1,
est -COOR⁵, -P(OR⁶)(OR⁷), -Si(OR⁵) (OR⁵) (OR⁵), SiR⁵R⁵R⁵ ou le groupe 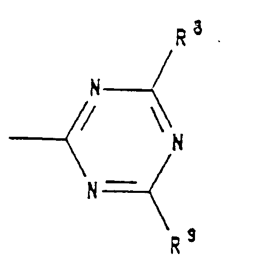
A lorsque n = 2
est -CO-, =POR⁵, =Si(OR⁵)(OR⁵), =SiR⁵R⁵ ou le groupe 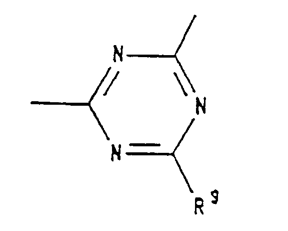
A lorsque n = 3
est ≡Si(OR⁵), ≡SiR⁵ ou le groupe 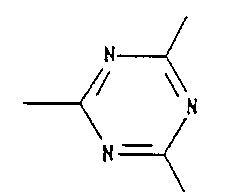
R⁵ représente un groupe alkyle en C₁-C₁₈, un groupe alcényle en C₂-C₁₈, un groupe alcynyle en C₂-C₁₈, un groupe cycloalkyle en C₅-C₁₂, un groupe bicycloalkyle en C₆-C₁₀, un groupe cycloalcényle en C₅-C₈, un groupe aryle en C₆-C₁₀, un groupe aralkyle en C₇-C₉, un groupe alkaryle en C₇-C₉, éventuellement substitué par alkyle en C₁-C₄ ou phényle, plusieurs restes R⁵ pouvant être identiques ou différents,
R⁶ et R⁷ sont identiques ou différents et représentent un groupe alkyle en C₁-C₁₈, un groupe alcényle en C₂-C₁₈, un groupe alcynyle en C₂-C₁₈, un groupe cycloalkyle en C₅-C₁₂, un groupe bicycloalkyle en C₆-C₁₀, un groupe cycloalcényle en C₅-C₈, un groupe aryle en C₆-C₁₀, un groupe aralkyle en C₇-C₉ ou un groupe aralkyle en C₇-C₉ substitué par des groupes alkyle en C₁-C₄ ou phényle, ou représentent un reste de formule II ou III 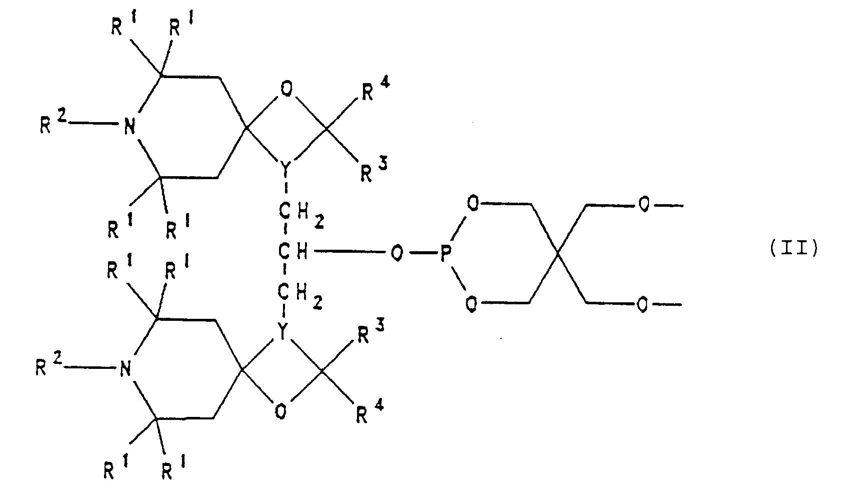 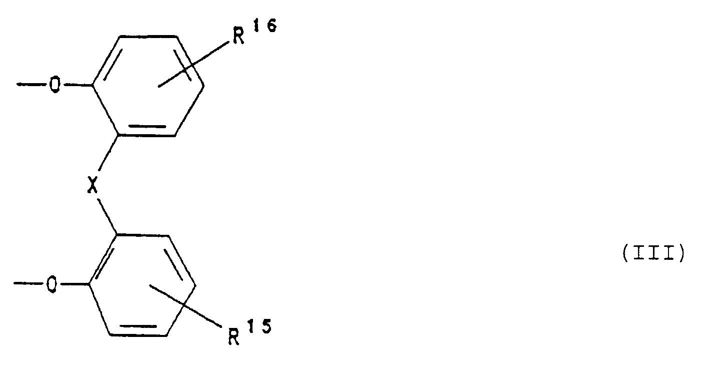 dans lesquelles R¹, R², R³, R⁴ et R⁵ ont les significations données ci-dessus, et X représente une liaison directe ou un groupe méthylène ou un groupe alkylidène 1,1 avec 2 à 5 atomes de carbone,
R⁸ et R⁹ sont identiques ou différents et représentent -OR⁵, NHR⁵, -NR¹¹R¹² ou l'un des deux groupes 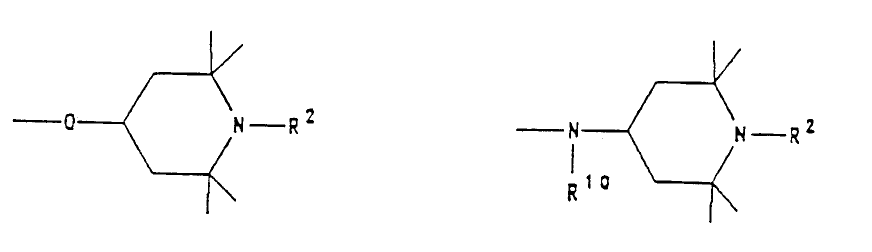 dans lesquels R¹⁰ représente un atome d'hydrogène, un groupe alkyle en C₁-C₁₂, un groupe cycloalkyle en C₅-C₇, un groupe aralkyle en C₇-C₉, un groupe alcanoyle en C₈-C₁₈, un groupe alcénoyle en C₃-C₅ ou un groupe benzoyle, ou 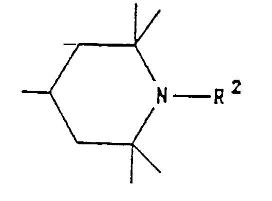
R¹¹ et R¹² sont identiques ou différents et représentent un groupe alkyle en C₁-C₁₀, un groupe cycloalkyle en C₅-C₇, ou un groupe aryle en C₆-C₁₀, ou R¹¹ et R¹² ensemble avec l'atome d'azote auquel ils sont liés, forment un cycle à 5 à 12 chaînons, qui peut encore contenir de l'oxygène.

2. Composés selon la revendication 1, caractérisés en ce que R¹ dans la formule I est un groupe méthyle.

3. Composés selon la revendication 1, caractérisés en ce que R² dans la formule I est un groupe méthyle.
